Europäisches Patentamt

European Patent Office

Office européen des brevets

(19)

(11) Publication number: **0 294 160**
**A1**

(12) **EUROPEAN PATENT APPLICATION**

(21) Application number: **88304962.9**

(22) Date of filing: **01.06.88**

(51) Int. Cl.⁴: **A 61 K 45/02**

(30) Priority: **02.06.87 US 57584**

(43) Date of publication of application:
**07.12.88 Bulletin 88/49**

(84) Designated Contracting States: **ES GR**

(71) Applicant: **SCHERING CORPORATION**
**2000 Galloping Hill Road**
**Kenilworth, New Jersey 07033 (US)**

(72) Inventor: **Meschievitz, Carlton K.**
**123 Ashwood Avenue**
**Summit New Jersey 07901 (US)**

**Stritar, Jeff A.**
**14 Settlers Lane**
**Westfield New Jersey 07090 (US)**

(74) Representative: **Ritter, Stephen David et al**
**Mathys & Squire 10 Fleet Street**
**London EC4Y 1AY (GB)**

(54) Treatment of chronic type b hepatitis with a combination of recombinant human alpha and gamma interferons.

(57) Hepatitis B virus replication is inhibited by treatment with a combination of human alpha interferon and human gamma interferon, administered either sequentially or concurrently; and the preferred alpha interferon is recombinant DNA human alfa-2b interferon, the preferred gamma interferon is recombinant DNA human gamma interferon and the preferred means of administration to a patient is by injection.

EP 0 294 160 A1

## Description

# TREATMENT OF CHRONIC TYPE B HEPATITIS WITH A COMBINATION OF RECOMBINANT HUMAN ALPHA AND GAMMA INTERFERONS

### Background

1. Field of Invention

This invention relates to a method of treating chronic type B hepatitis by inhibiting replication of the hepatitis B virus with a combination of (a) human gamma interferon (hIFN-γ) sometimes referred to as human immune interferon and (b) human alpha interferon (hIFN-α) sometimes referred to as human lenkocyte interferon, which, in combination, display greater inhibitory effects against replication of the hepatitis B virus than is expected from their individual activities.

2. Prior Art

The interferons are a group of proteins with diverse antiviral, immunomodulatory and antiproliferative effects. They are separated into three classes based on their antigenicity and cell of origin, i.e. alpha (leukocyte), beta (fibroblast) and gamma (immune) interferons. Alpha and beta interferons are called type I interferons. Their genes have sequence homology and are located on the same chromosome. They also share a common cell receptor.

Although beta interferon is contemplated as suitable for use in combination with gamma interferon in this invention, human alpha interferon is preferred with recombinant DNA derived human alpha interferon most preferred. The invention will be described referring to such alpha interferon.

Human alpha interferon is a naturally occurring mixture of at least eleven compounds including those designated alpha-1 interferon and alpha-2 interferon. Alpha interferon exhibiting biological properties similar to those of naturally occurring human leukocyte interferon can be made by recombinant methods.

A number of alpha interferon species or components are known and are usually designated by a numeral after the Greek letter alpha, and all are contemplated for use in this invention, thus, the species designated "human alpha-1 interferon" is contemplated for use in this invention as is "human alpha-2 interferon" which under USAN is designated Interferon Alfa-2b and is the preferred species of recombinant DNA human alpha interferon for use in this invention. Also suitable is recombinant DNA human interferon alfa-2a which can be made as disclosed in Rubenstein, Biochem. Biophys, Acta, 695, 5-16 (1982).

Human interferon alfa-2b can be produced in bacteria using recombinant techniques. In addition, human interferon alfa-2b may be prepared, by recombinant-DNA methods disclosed by Nagata et al. Nature, 284, 316-320 (1980), European Patent 32,134 and U.S. Patent No. 4,289,690. Various alpha interferon species are disclosed in U.S. patent 4,503,035. The preferred human interferon alfa-2b used in this invention is also denoted herein as "hIFN-α2b".

Gamma interferon has a number of characteristics known in the art that differentiate it from alpha and beta interferons. It is called a Type II interferon, its gene has no homology to the alpha and beta interferon genes and is on a separate chromosone. Gamma interferon also has a separate cell receptor. In addition, human gamma interferon has higher levels of immunomodulatory activity. Human gamma interferon may be produced by T-lymphocytes stimulated by mitogens or antigens to which they are sensitized. It may also be obtained through cloning and expression techniques now well known in the art.

Although the source of the human gamma interferon used in this invention is not critical, it is required that such hIFN-γ be a high purity material that is not contaminated by cell constituents or cell debris of the interferon-expressing cell. A preferred hIFN-γ used in this invention is produced by recombinant DNA technology and then purified as taught in Japanese Patent Application No. 281376, filed December 27, 1984 and foreign counterparts thereof, e.g. PCT International Publication No. 8604067 published July 17, 1986. The purification process comprises adding one or more salts of zinc or copper and polyethyleneimine in the extraction. More particularly, it comprises suspending the culture cells of a recombinant microorganism in a buffer solution containing one or more salts of zinc or copper, e.g. zinc chloride, zinc sulfate, zinc acetate, zinc acetylacetonate and copper sulfate, in a range of about 0.5-5 mM in the case of zinc salts and 0.01-3 mM in the case of copper salts, disrupting the cells, then adding poyethyleneimine to the centrifuged supernatant, e.g. to a final concentration of 0.5-1.1%, and subsequently purifying by a conventional method, e.g. combining several chromatographic methods and dialysis.

Human gamma interferon can be made, for example, by the procedures disclosed in Gray, et al., Nature, 295,503-508 (1982) and Epstein, Nature, 295, 453-54 (1982).

To date, much has been learned about the effect of the interferons on viruses.

Alpha interferons have been shown to inhibit replication of the hepatitus B virus by Scullard et al, J. Infect. Dis., 143, 772-783 (1981). Prolonged therapy resulted in remissions of disease in 20% to 50% of patients as disclosed by Scullard et al, supra; Lok et al, Hepatology, 3, 865 (1983). Dooley et al., Gastroenterology, 90; 150-57 (1986); and Hoofnagel et al. Hepatology, 5, 1033 (1985).

Hoofnagle et al., supra, also reported that in a randomized controlled trial of a four month course of recombinant interferon alfa-2b conducted in patients with chronic type B hepatitis, remissions occurred in 32% of treated patients and 14% of untreated controls.

In some instances the combined use of human alpha-2 interferon and gamma interferon resulted in inhibiting replication of some viruses as reported by Fleischmann et al., J. Interferon Res. 4, 265-74 (1984) and Antiviral Research 4, 357-60 (1984), but hepatitis B virus is not included in the discussion.

The treatment of chronic hepatitis B with alpha interferon combined with an antiviral drug such as adenine arabinoside monophosphate in selected patients is reported in World Health Organization Technical Report Series 676; "Interferon Therapy" (1982)

## SUMMARY OF THE INVENTION

This invention relates to a method of treating chronic type B hepatitis by inhibiting replication of the hepatitis B virus with a combination of recombinant DNA human gamma interferon and recombinant DNA human alpha interferon (preferably hIFN-α2b) sufficient to inhibit replication of the hepatitis B virus. When the hepatitis B virus is in an animal, including humans, the preferred mode of administration is parenteral, i.e. intravenous, intramuscular, subcutaneous and intraperitoneal, although other administration modes, such an enteral, e.g. rectal or oral, and topical, e.g. nasal, transdermal and the like, can be used. The two interferons can be administered concurrently or sequentially.

This invention provides compositions and methods for treating chronic type B hepatitis and/or inhibiting replication of the hepatitis B virus utilizing lower doses of recombinant human gamma interferon and recombinant human alpha interferon, preferably human interferon alfa-2b, in combination than required if each is used alone.

In addition, this invention provides a method of treating chronic type B hepatitis by administering to a patient in need of such treatment a pharmaceutical composition comprising a sufficient amount of recombinant human gamma interferon and recombinant human alpha interferon in combination in a pharmaceutically acceptable composition to be effective in inhibiting replication of the hepatitis B virus. The invention also provides a method of inhibiting replication of the hepatitis B virus by treating such virus with a replication inhibiting amount of a combination of recombinant human gamma interferon and recombinant human alpha interferon. The active ingredients can be administered separately in any effective sequence, or concurrently.

Administration of the compositions or combinations of this invention can be via any of the accepted modes of administration for interferons. These methods include parenteral, topical, depot and transdermal. Subcutaneous or intravenous injection is preferred.

Depending on the intended mode of administration, the compositions containing the gamma and/or alpha interferons may be in the form of solid, semi-solid or liquid dosage forms, preferably for example, powders for reconstitution. Also the interferons may be in the form of tablets, pills, capsules, liquids, suspensions, or the like, preferably in unit dosage forms suitable for single administration of precise dosages.

The pharmaceutically acceptable carriers and excipients useful in this invention are conventional to the formulator skilled in this art. There may be included other medicinal and pharmaceutical agents. Excipients which may be included, can be, but are not limited to, other proteins, such as, human serum albumin or plasma preparations.

Liquid injectable pharmaceutically acceptable and administerable compositions can, for example, be prepared by dissolving, dispersing, etc. the interferons in a carrier, such as, for example, water, saline, aqueous dextrose, glycerol, ethanol, and the like, to thereby form a solution or suspension for injection. If desired, the pharmaceutical composition to be administered may also contain minor amounts of nontoxic auxiliary substances such as wetting or emulsifying agents, preservatives, pH buffering agents and the like, for example, sodium acetate or sorbitan monolaurate. Actual methods of preparing such dosage forms are known, or will be apparent, to those skilled in this art; for example, see Remington's Pharmaceutical Sciences, Mack Publishing Company, Easton, Pennsylvania, 15th Edition, 1975. The composition or formulation to be administered will, in any event, contain a quantity of the active component(s) in an amount effective to achieve the desired effect in the subject being treated.

Based on the judgment of the attending clinician, the amount of active compounds administered will, of course, be dependent on the subject being treated, the severity of the hepatitis B infection, the patients tolerance to treatment, the manner of administration and other conventional criteria.

The combination of gamma interferon and alpha interferon can contain a wide range of international reference units per ml (IRU/ml) of each interferon and still display a greater than additive effect against hepatitis B virus, for examples, the amount of gamma interferon administered can be from about 5,000 to 1 million international reference units per day and the amount of alpha interferon administered can be from about 100,000 to 10 million international reference units per day. A preferred dosage is that amount of each interferon which results in a 10% to 30% decrease in serum DNA-polymerase (DNA-p) levels compared to such levels prior to treatment when each interferon is administered alone. Those amounts should result in a decrease of greater than 60% of the serum DNA-p levels when the interferons are used in combination to treat a patient.

The combination of interferons used according to this invention should exhibit greater than additive effects against hepatitis B virus.

## DETAILED DESCRIPTION

The following is an illustration of the clinical protocol utilized in practicing this invention.

## Materials and Methods

In order to measure the separate and greater than additive effects of recombinant DNA human alfa-2b interferon and recombinant DNA human gamma interferon on chronic type B hepatitis, the DNA-p (DNA-polymerase) blood serum levels are measured before and after treatment. The measurements are made by conventional test procedures as described in Berringer et al. J. of Medical Virology 9, 57-68 (1982) and Kaplan, et al., J. of Virology. 12, 995-1002 (1973). The blood serum levels prior to treatment are compared to the post treatment levels with a decrease indicating amelioration of the infection.

Interferons

The hIFN-$\alpha$2b used is supplied as a lyophilized powder in vials containing $5 \times 10^6$ and $1 \times 10^7$ international units (IU). The hIFN-$\gamma$ used is supplied in vials containing $2 \times 10^5$ and $2 \times 10^6$ IU. 1.0 ml of sterile, non bacteriostatic water for injection is added to each vial. The resulting reconstituted solution is kept under refrigeration to maintain stability.

Treatment

Patients with chronic type B hepatitis are administered by subcutaneons injection a combination of recombinant human gamma interferon and recombinant human alfa-2b interferon for a period of time sufficient to lower their blood serum levels of DNA-p to the extent needed to indicate the hepatitis B virus has stopped replicating and has disappeared from the blood. The amount of each of the interferons used and the dosage regimen is determined in the judgment of the attending clinician, taking into consideration the patient's condition, tolerance to treatment and efficacy of the drugs and the severity of the infection. Generally the dosages are from 5,000 to 1 million international units of recombinant human gamma interferon, and 100,000 to 10 million international units of recombinant human alfa-2b interferon either daily or every other day, depending on the clinicians judgment. Efficacy is measured by serum hepatitis B levels and DNA-p suppression. Tolerance is measured by side effects.

In order to ascertain whether greater than additive effects are obtained, control patients are treated with recombinant human gamma interferon alone, then recombinant human alfa-2b interferon alone at the dosages described above. Then the patients are treated with a combination of the two interferons at levels of each which produced about a 10% to 30% DNA-p reduction in such patients when administered alone.

The results of the combined administration should result in an effect which is greater than additive.

## Claims

1. A method of inhibiting replication of the hepatitis B virus which comprises treating the virus with an effective replication inhibiting amount of a combination of human alpha interferon and human gamma interferon either sequentially or concurrently.

2. A method of claim 1 wherein said human alpha interferon is recombinant DNA human alpha interferon.

3. A method of claim 2 wherein said human gamma interferon is recombinant DNA human gamma interferon.

4. A method of claim 2 wherein the alpha interferon is alfa-2b interferon.

5. A method of claim 4 wherein the application of said interferons is sequential.

6. A method of claim 4 wherein the application of said interferons is concurrent.

7. A method of treating chronic hepatitis B which comprises administering to a patient in need of such treatment an effective amount of a combination of human gamma interferon and human alpha interferon, either sequentially or concurrently.

8. A method of claim 7 wherein said human alpha interferon is recombinant DNA human alpha interferon.

9. A method of claim 8 wherein said human gamma interferon is recombinant DNA human gamma interferon.

10. A method of claim 9 wherein the alpha interferon is alfa-2b interferon.

11. A method of claim 10 wherein the administration of the interferons is sequential.

12. A method of claim 10 wherein the administration of the interferons is concurrent.

13. A method of claim 7 wherein the administration of the interferons is by injection.

14. Use of human alpha interferon and human gamma interferon in the preparation of a medicament for treating chornic hepatitis B.

15. Human alpha interferon in a pharmaceutical composition for the treatment of chronic hepatitis B in conjunction with human gamma interferon.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 107 498  (GENENTECH)<br>* Claims 1,6 *<br>--- | 1,2,3 | A 61 K  45/02 |
| X | JOURNAL OF BIOLOGICAL RESPONSE MODIFIERS, vol. 6, no. 2, 1987, pages 141-153, Raven Press, New York, US; H.R. HUBBELL et al.: "Synergistic antiproliferative effect of recombinant alpha-interferons with recombinant gamma-interferon"<br>* Pages 141,147 *<br>--- | 1,2,3 | |
| X | INFECTION AND IMMUNITY, vol. 40, no. 1, April 1983, pages 35-38, American Society for Microbiology, Washington, D.C., US; D.A. WEIGENT et al.: "Potentiation of lymphocyte natural killing by mixtures of alpha or beta interferon with recombinant gamma interferon"<br>* Page 35 *<br>----- | 1,2,3 | |

**TECHNICAL FIELDS SEARCHED (Int. Cl.4)**

A 61 K

The present search report has been drawn up for all claims

| Place of search | Date of completion of the search | Examiner |
|---|---|---|
| THE HAGUE | 19-09-1988 | PEETERS J.C. |